# EUROPEAN PATENT APPLICATION

(11) **EP 4 253 538 A1**
(43) Date of publication of application: **04.10.2023**
(21) Application number: 21898742.8
(22) Date of filing: 30.11.2021
(51) Int. Cl.: C12N 15/10, C12Q 1/6806

(54) **METHOD FOR ISOLATING NUCLEIC ACID USING BINDING BUFFER INCLUDING COMPOUND HAVING LOW OR INTERMEDIATE DIELECTRIC CONSTANT**

(30) Priority: 30.11.2020 KR 20200164685
(71) Applicant: Bioneer Corporation, Daejeon 34302 (KR)
(72) Inventor: PARK, Han-Oh, Sejong-si 30151 (KR); KIM, Myung Il, Daejeon 35247 (KR); PARK, Sang Ryoung, Daejeon 34048 (KR); LEE, Na Young, Daejeon 34016 (KR); GU, Geon Woo, Daejeon 34009 (KR)
(74) Representative: Mewburn Ellis LLP
(86) International application number: PCT/KR2021/017860
(87) International publication number: WO 2022/114917

(57) **Abstract**

The present invention relates to a method for isolating a nucleic acid by using: a binding buffer containing acetic acid or phosphoric acid; and the pH difference between binding and washing buffers, and an elution buffer, wherein silica magnetic particles are added to a biological sample including a nucleic acid to which a binding buffer containing acetic acid, phosphoric acid, etc., has been added, thereby binding the nucleic acid to the magnetic particles, after which a magnetic field is applied to separate the magnetic particles, and the nucleic acid is purified in a basic pH elution buffer. The method uses the pH difference, and can isolate the nucleic acid more quickly and efficiently than centrifugation or gravity separation. In addition, since ethanol is not used, high-purity nucleic acid can be isolated and purified to obtain more accurate results in subsequent experiments.

## Description

### [Technical Field]

The present invention relates to a method of isolating a nucleic acid using an acidic binding buffer including a compound having a lower dielectric constant (or relative permittivity) than ethanol and also using a change in pH of the binding buffer, a washing buffer, and an elution buffer, and more particularly to a method of isolating a nucleic acid including dissolving a biological sample including a nucleic acid using a lysis buffer, adding an acidic binding buffer including a compound having a lower dielectric constant than ethanol and silica magnetic particles to allow the nucleic acid to bind to the magnetic particles, performing washing with a washing buffer at a low pH, separating the magnetic particles by applying a magnetic field, and purifying the nucleic acid in an elution buffer at a high pH.

### [Background Art]

Methods of rapidly isolating and purifying high-purity nucleic acids from biological materials are important in biological research based on molecular biology and biochemistry and in diagnostic processes for diagnosing diseases or confirming infection. To this end, various studies have been conducted on methods of selectively, effectively, and reproducibly isolating nucleic acids from various types of materials contained in cell lysates. Typically acceptable nucleic acid isolation techniques have to satisfy conditions that impurities should not be included, isolation efficiency should be high, and the structures or properties of nucleic acids should not be changed during the isolation process.

In order to isolate nucleic acids from biological materials, cells are lysed, only desired nucleic acids are isolated from the cell lysate, and finally nucleic acids are eluted. Existing nucleic acid isolation methods include methods using phenol/chloroform, methods using silica resin, methods using affinity resin, ion exchange resin chromatography methods, and methods using magnetic particles that have emerged recently. These are described in U.S. Patent No. 4,923,978, U.S. Patent No. 5,057,426, U.S. Patent No. 7,173,124, European Patent No. 0796327, etc., and are mainly methods in which a specific solvent is used to isolate a nucleic acid bound to a solid support.

Examples of currently useful nucleic acid isolation methods include a method using a filter column and a method using magnetic particles. Commonly, a chaotropic salt such as guanidine hydrochloride is added in the binding step, whereby hydrogen bonds of nucleic acids are broken, and nucleic acids, the surfaces of which are negatively charged, are allowed to bind to magnetic particles or silica on the surface of the column filter by electrostatic attraction. Nucleic acid isolation using the filter column, which is the most popular, is of QIAGEN's spin column type, and a process of pressurization or centrifugation is essential, during which the nucleic acid binds to the entire surface area of the filter in the column. Thereafter, ethanol treatment is performed before eluting the nucleic acid, the column is completely dried, and then the nucleic acid is eluted using a certain amount of a nucleic acid elution solution. However, the filter column method is disadvantageous in that, when a high-concentration nucleic acid is required, efficient nucleic acid isolation is difficult because only a small amount of elution solution is incapable of eluting the entire nucleic acid bound to the column. Also, upon nucleic acid isolation, this method requires processes such as centrifugation or pressurization, which makes it difficult to apply to diagnostic automation that requires simultaneous isolation of many different biological samples.

The method using magnetic particles is performed in a manner in which magnetic particles are added to a cell mixture to induce binding between nucleic acids or proteins and magnetic particles and only nucleic acids or proteins bound to magnetic particles are selectively isolated and purified using an external magnetic field. A method of isolating plasmid DNA from genomic DNA using magnetic particles (U.S. Patent No. 5665554) is disclosed, and a method of isolating a target material including forming a complex of a biological target material and silica magnetic particles using silica magnetic particles and then isolating the target material using a magnetic field (U.S. Patent No. 6027945) has already been reported.

Therefore, thorough research into isolating nucleic acids or proteins from cell mixtures using magnetic particles is ongoing (U.S. Patent No. 9,163,272, WO2015/088201), and it is difficult to automate conventional isolation methods, but use of magnetic particles makes it easy to apply an automated system and thus a system that automates purification and isolation of nucleic acids or proteins using magnetic particles has already been developed (Korean Patent No. 1555534, Korean Patent No. 1443727). The method using magnetic particles includes allowing nucleic acids and magnetic particles to bind to each other by adding magnetic particles to a cell mixture and then selectively isolating only the nucleic acids bound to the magnetic particles using an external magnetic field. Here, it is known that the size of magnetic particles used to selectively isolate and purify only the nucleic acid is appropriately hundreds of nanometers, but recently, techniques using small magnetic particles having a size of tens of nanometers or less have been reported.

In order to quickly isolate nucleic acids bound to magnetic particles from biological samples, various products for applying an external magnetic field more easily are also being released. There is exemplified a MagListo^{™} Magnetic Separation Rack (Bioneer) including a tube configured to inject a biological sample, a tube fixer configured to fix the tube, and a magnet fixer configured to apply a magnetic field at an external position corresponding to the tube. As such, the magnet fixer may be designed to be removably attached to the tube fixer with a magnet, so that the user may easily isolate nucleic acids or proteins from biological materials.

However, the method using the magnetic particles has a disadvantage of difficulty in obtaining high-concentration nucleic acids required for molecular diagnosis. After using a washing solution including ethanol in the nucleic acid isolation process, ethanol remaining in the magnetic particles must be completely removed in order to prevent inhibition of polymerase chain reaction (PCR), which is the most typical purpose of nucleic acid extraction. Hence, a process of drying the magnetic particles is absolutely necessary, which inevitably increases the time required for extraction. Moreover, in the case in which ethanol is not completely removed in this process, PCR cannot be performed, making it impossible to obtain accurate results and losing biological samples.

In order to alleviate the above-mentioned disadvantages of magnetic particles, U.S. Patent No. 6,914,137 discloses a method of extracting nucleic acids and other biological molecules from biological samples, particularly blood, by bringing biological molecules into contact with a solid at a first pH so that the biological molecules bind to the solid and extracting the biological molecules bound to the solid by elution using an elution solvent at a second pH, and U.S. Patent No. 8,067,579 also discloses a method in which a solid support is positively charged so that a nucleic acid may easily bind to the solid support, and also in which a negative charge environment is created so that the nucleic acid is easily eluted.

In addition, the present inventors paid attention to the dielectric constant level of the compound mixed in the nucleic acid binding buffer. The dielectric constant is a measure of charge separation within a material. For a material that may be ionized, such as acetic acid, which is used most in the present invention, actual intramolecular charge separation may be greater than that of alcohol-based compounds that are hardly ionized. The present inventors referred to this dielectric constant value to search for other materials that may be used as binding buffers besides ethanol. The dielectric constant of ethanol or isopropanol, which is typically used as a reagent for nucleic acid binding, was searched, and several candidate materials having a lower dielectric constant and not harmful enough to be used for diagnosis were selected and used in experimentation. This numerical value was not considered or validated as an absolute measure of the extent of nucleic acid binding, and was used only as a reference for searching for compounds to be used for the binding buffer.

Therefore, the present inventors have found a phenomenon in which a nucleic acid binds to silica magnetic particles in an acidic binding buffer and an acidic washing buffer at a concentration below or above a certain level using typical silica magnetic particles and the nucleic acid is eluted in an elution buffer at a high pH, and ascertained that the nucleic acid may be isolated quickly and efficiently based on such a phenomenon, and also, ethanol is not used and thus more accurate results may be obtained in subsequent experiments, culminating in the present invention.

### [Disclosure]

It is an object of the present invention to provide a nucleic acid extraction method capable of isolating a nucleic acid from a biological sample more quickly and efficiently than before using a difference in pH between buffers.

It is another object of the present invention to provide a kit for nucleic acid isolation and purification developed by applying the nucleic acid extraction method using a difference in pH between buffers.

In order to accomplish the above objects, the present invention provides a method of isolating a nucleic acid using a difference in pH between buffers, comprising: (a) binding a nucleic acid to silica magnetic particles by adding silica magnetic particles to a sample containing a nucleic acid, a mixture of binding buffer comprising 0.175 M or more aqueous acetic acid solution or 2.18 M or less aqueous phosphoric acid solution; (b) washing the magnetic particles to which the nucleic acid binds in an acidic pH buffer; and (c) separating the magnetic particles and eluting the nucleic acid in a basic pH elution buffer.

In addition, the present invention provides a kit for isolating and purifying a nucleic acid using the nucleic acid extraction method using a difference in pH between binding, washing, and elution buffers.

### [Description of Drawings]

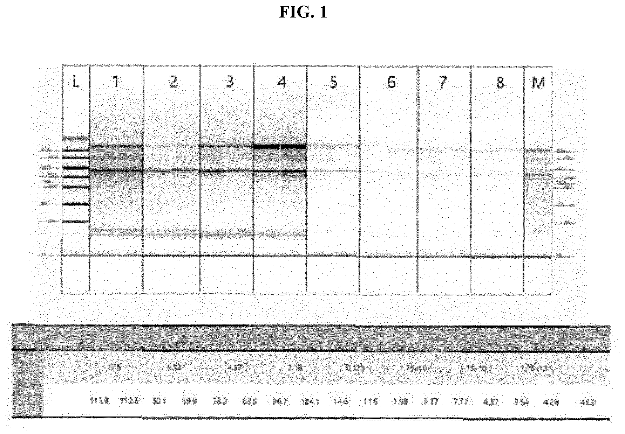

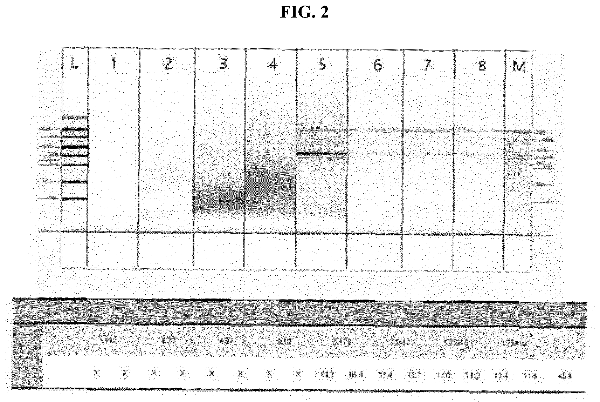

FIGs. 1 and 2 show extracting total RNA from subcultured HeLa cells and analyzing the same using an Agilent 5200 Fragment Analyzer System in order to determine effectiveness of gene extraction when using a binding buffer including an acidic compound selected in the present invention;

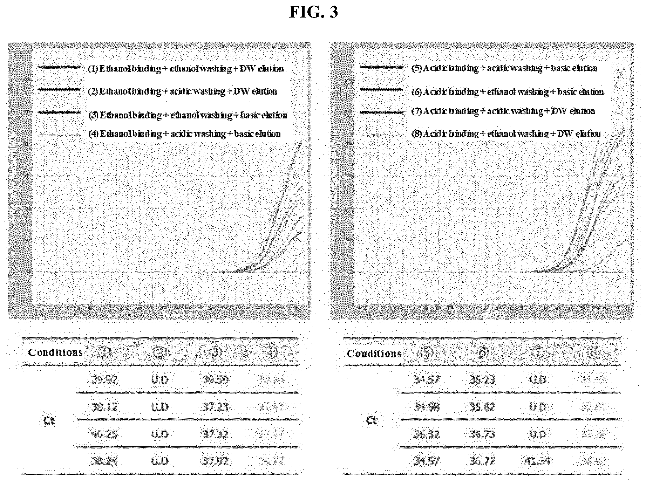

FIG. 3 shows an experiment to verify validity of the acidic binding-acidic washing-basic elution hypothesis using various compositions of binding, washing, and elution buffers, in which results of analysis of real-time reverse transcription polymerase chain reaction (real-time RT-PCR) after extraction and purification of a viral nucleic acid using a total of eight buffer combinations are shown in graphs and tables;

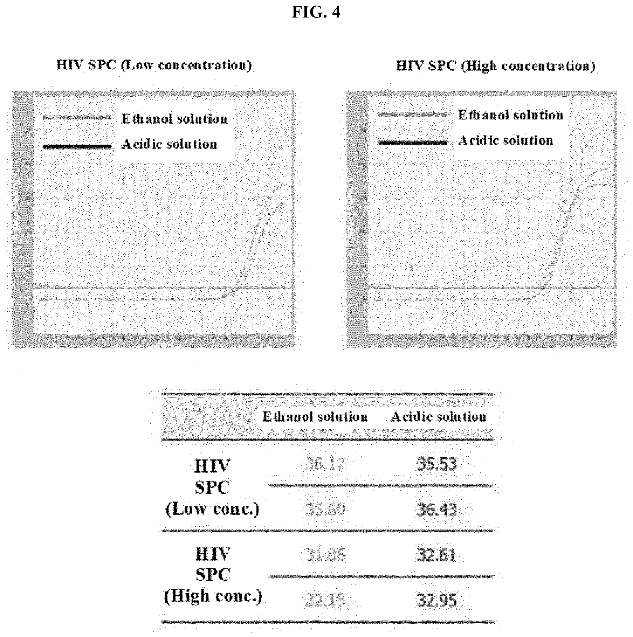

FIG. 4 shows an experiment to confirm the equivalence of gene extraction performance of the conventional kit and the inventive solution composition, in which results of analysis of real-time reverse transcription polymerase chain reaction (real-time RT-PCR) after purification of a viral nucleic acid using two binding buffers are shown in graphs and a table; and

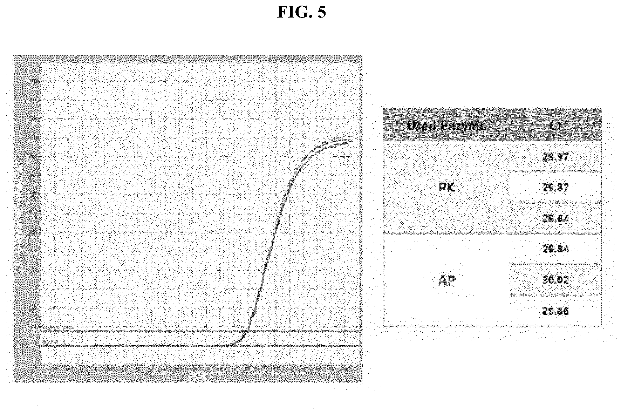

FIG. 5 shows an experiment to confirm the extraction efficiency upon sample dissolution using an acidic protease as compared with proteinase K, in which results of analysis of real-time reverse transcription polymerase chain reaction after purification of a viral nucleic acid using two enzymes are shown in a graph and a table.

### [Mode for Invention]

Unless otherwise defined, all technical and scientific terms used herein have the same meanings as typically understood by those skilled in the art to which the present invention belongs. In general, the nomenclature used herein is well known in the art and is typical.

The present invention uses similarity with conventional ethanol binding technology, and with reference to the dielectric constant table, candidate materials that are less harmful and are acidic and thus suitable for use in a binding buffer were selected. A biological sample containing a nucleic acid is added to the searched binding buffer and silica magnetic particles to allow the nucleic acid to bind, a magnetic field is applied to separate the magnetic particles, and the nucleic acid is purified in an elution buffer at a high pH, whereby the nucleic acid may be isolated by a pH change, and moreover, the nucleic acid may be isolated quickly and efficiently without the need to use ethanol that is a nucleic acid washing solution and a centrifuge by isolating the nucleic acid using a magnetic field.

Accordingly, an aspect of the present invention pertains to a method of isolating a nucleic acid using a difference in pH between buffers, comprising: (a) binding a nucleic acid to silica magnetic particles by adding silica magnetic particles to a sample containing a nucleic acid, a mixture of binding buffer comprising 0.175 M or more aqueous acetic acid solution or 2.18 M or less aqueous phosphoric acid solution; (b) washing the magnetic particles to which the nucleic acid binds in an acidic pH buffer; and (c) separating the magnetic particles and eluting the nucleic acid in a basic pH elution buffer.

In the present invention, the isolation method does not use an alcohol precipitation method.

In the present invention, the magnetic particles used for nucleic acid extraction have a particle size of 50 nm to 1 µm, and are magnetic particles widely used by ordinary technicians to isolate and purify nucleic acids, rather than special magnetic particles that must be synthesized in a complicated manner. In particular, *AccuNanoBead*^{™} manufactured by Bioneer may be used, but the present invention is not limited thereto.

In the present invention, the magnetic particles may be coated with a material containing a siliceous material selected from the group consisting of silica, quartz, and silicon.

The nucleic acid is preferably DNA (deoxyribonucleic acid) or RNA (ribonucleic acid), and examples thereof may include, but are not limited to, genomic DNA, plasmid DNA, phage DNA, recombinant DNA, mRNA, rRNA, tRNA, recombinant RNA, micro RNA, and the like.

In the present invention, a change in pH of the binding buffer, the acidic pH buffer, and the basic pH elution buffer may be used.

A binding buffer including an acidic compound such as acetic acid and magnetic particles are added to the biological sample containing the nucleic acid to allow the nucleic acid to bind. Thereafter, only the supernatant is removed using a magnet, followed by washing with an acidic washing buffer, removal of the supernatant using a magnet, and then dissolution of the magnetic particles to which the nucleic acid binds in a basic elution buffer to collect the nucleic acid.

In the present invention, steps (b) and (c) may include applying a magnetic field using a magnet.

In the present invention, step (a) may include mixing the sample including the nucleic acid with an acidic buffer at a pH of 1 to 6 and adding magnetic particles thereto to allow the nucleic acid to bind to the magnetic particles.

In the present invention, step (b) may include attaching the magnetic particles using a magnet, removing the supernatant, and performing washing with an acidic solution at a pH of 4 to 6.5.

In the present invention, step (c) may include removing the supernatant using a magnet and dissolving the magnetic particles to which the nucleic acid binds in a basic solution at a pH of 8.0 to 10.0 to collect the nucleic acid.

In the present invention, the mixture of the binding buffer may further include an acidic protease or proteinase K.

In addition, another aspect of the present invention pertains to a kit for isolating and purifying a nucleic acid using the nucleic acid extraction method based on a difference in pH between buffers. Preferably, the kit further uses an acidic protease and/or proteinase K, thus dissolving a sample in an acidic lysis buffer and extracting a nucleic acid based on a difference in pH between the buffers.

A better understanding of the present invention may be obtained through the following examples. These examples are merely set forth to illustrate the present invention, and are not to be construed as limiting the scope of the present invention, as will be apparent to those skilled in the art.

### [Examples]

### Example 1: Comparison of nucleic acid extraction efficiency depending on concentration of acetic acid or phosphoric acid contained in binding buffer (total RNA extraction from HeLa cells and analysis using fragment analyzer)

In order to confirm effectiveness of acetic acid and phosphoric acid selected as candidate materials for use in a binding buffer, nucleic acid extraction efficiencies were compared when acetic acid and phosphoric acid were used to prepare an acidic solution and also when the concentrations thereof were varied. Total RNA from HeLa cells was purified using a binding buffer including each of two acids and then analyzed using an Agilent 5200 Fragment Analyzer System. As a comparative group, a MagListo^{™} 5M Universal RNA Extraction Kit (Bioneer, K-3613) was used.

As a biological sample, 1,000,000 cells were counted and used by subculturing Hela cells (distributed from SiRNAgen Therapeutics). For cell subculture, MEM Eagle with EBSS and L-glutamine (Lonza, 12-51 1F), fetal bovine serum (WELGENE, S001-01), DPBS (Dulbecco's phosphate-buffered saline, WELGENE, LB 001-01), and TrypLE^{™} Express (Gibco, 12605-010) were used and culture was carried out in a carbon dioxide incubator at 37°C.

A buffer including proteinase K and guanidine hydrochloride was added to the cells counted as above and mixed, after which the cells were lysed at 60°C. The cell lysate was mixed with an acidic solution at each concentration and silica magnetic particles, so that the nucleic acid and the magnetic particles were allowed to bind to each other. After removing only the supernatant using a magnet, washing was performed once again using a low-concentration acidic solution (at a pH of 4.5-5.0). After removing the supernatant using a magnet, the silica magnetic particles to which the nucleic acid bound were dissolved in 100 µl of a Tris hydrochloride solution (at a pH of 9.0-10.0) to collect the nucleic acid.

The collected nucleic acid solution was analyzed using an Agilent 5200 Fragment Analyzer System. The reagent used was a Fragment Analyzer RNA Kit (Agilent, DNF-471).

As shown in FIGs. 1 and 2, it was confirmed that both acetic acid and phosphoric acid were effective as the compound to be added to the binding buffer for nucleic acid binding and also that cell nucleic acid extraction performance thereof at a specific concentration was equivalent to that of the method using an ethanol solution.

### Example 2: Comparison of extraction efficiency depending on composition of binding, washing, and elution buffers (viral nucleic acid extraction and real-time polymerase chain reaction)

In order to search for the optimal composition and verify the content of the invention, nucleic acid extraction efficiencies were compared using buffers having the compositions described below. Real-time reverse transcription polymerase chain reaction (real-time RT-PCR) was performed after purifying viral nucleic acids using 8 types of buffer combinations prepared using 2 types each of binding buffer, washing buffer, and elution buffer.

As a biological sample, normal human serum (Merck, S1-LITER) and HIV-1 (WHO International Standard 3rd HIV-1 International Standard, NIBSC, NIBSC code: 10/152) were used.

100 µl of serum was mixed with 1000 and 200 IU (international unit) of HIV-1, after which guanidine hydrochloride and proteinase K were added to the mixture and the cells were lysed at 60°C.

The binding buffer and silica magnetic particles were added to the cell lysate and mixed, so that the nucleic acid and the magnetic particles were allowed to bind to each other. After removing only the supernatant using a magnet, washing was performed once again using a washing buffer. After removing the supernatant using a magnet, the silica magnetic particles to which the nucleic acid bound were dissolved in 100 µl of an elution buffer to collect the nucleic acid. The binding buffer included ethanol or an acetic acid solution (12.5 v/v%). As the washing buffer, ethanol including a low-concentration basic buffer (at a pH of 8.0) or a low-concentration acidic solution (at a pH of 4.5-5.0) was used. As the elution buffer, DEPC-DW or a low-concentration Tris hydrochloride buffer (at a pH of 9.0-10.0) was used.

The collected nucleic acid solution was added to an *AccuPower*^{®} HIV-1 Quantitative RT-PCR Kit and real-time reverse transcription polymerase chain reaction was performed. After addition of 50 µl of the nucleic acid solution to the *AccuPower*^{®} HIV Quantitative RT-PCR Kit, the upper surface of the diagnostic kit was completely sealed using an adhesive sealing film, and the diagnostic kit composition and the nucleic acid were thoroughly mixed using an *ExiSpin*^{™} (Bioneer, BS-010211-AAL). Here, real-time RT-PCR was performed under reaction conditions shown in Table 1 below, and an *Exicycler*^{™} 96 (Bioneer, A-2060) was used for real-time nucleic acid amplification reaction.

As shown in FIG. 3, it was confirmed that the use of the acidic binding buffer, the acidic washing buffer, and the basic elution buffer for nucleic acid binding was vastly superior in view of viral nucleic acid extraction performance compared to when using all other compositions.

**[Table 1]**

| Line | STEP | Temperature | Running Time |
|---|---|---|---|
| 1 | cDNA synthesis | 50°C | 15 min |
| 2 | Pre-denaturation | 95°C | 5 min |
| 3 | Denaturation | 95°C | 5 see |
| 4 | Annealing & Extension | 55°C | 5 see |
| 5 | Scan | | |
| 6 | Go to line 3 Cycle 45 | | |
| 7 | End | | |

### Example 3: Comparison of nucleic acid extraction methods using ethanol solution and acidic solution (viral nucleic acid extraction and real-time polymerase chain reaction)

Using the existing viral nucleic acid extraction kit released by the present company (*AccuPrep*^{®} Viral RNA Extraction Kit, Bioneer, K-3033, referred to as an "ethanol solution") and the composition using an acidic buffer (referred to as an "acidic solution"), nucleic acid extraction efficiencies were compared.

As a biological sample, normal human serum (Merck, S1-LITER) and HIV-1 (WHO International Standard 3rd HIV-1 International Standard, NIBSC, NIBSC code: 10/152) were used.

100 µl of serum was mixed with 1000 and 200 IU (international unit) of HIV-1, after which guanidine hydrochloride and proteinase K were added to the mixture and the cells were lysed at 60°C.

A weakly acidic binding buffer including low-concentration acetic acid (12.5 v/v%) and silica magnetic particles were added to the cell lysate and mixed, so that the nucleic acid and the magnetic particles were allowed to bind to each other. After removing only the supernatant using a magnet, washing was performed once again using a weakly acidic low-concentration washing buffer (at a pH of 4.5-5.0). After removing the supernatant using a magnet, the silica magnetic particles to which the nucleic acid bound were dissolved in 100 µl of a low-concentration Tris hydrochloride buffer (at a pH of 9.0-10.0) to collect the nucleic acid.

The collected nucleic acid solution was added to an *AccuPower*^{®} HIV-1 Quantitative RT-PCR Kit and real-time reverse transcription polymerase chain reaction was performed. After addition of 50 µl of the nucleic acid solution to the *AccuPower*^{®} HIV Quantitative RT-PCR Kit, the upper surface of the diagnostic kit was completely sealed using an adhesive sealing film, and the diagnostic kit composition and the nucleic acid were thoroughly mixed using an *ExiSpin*^{™} (Bioneer, BS-010211-AAL). Here, real-time RT-PCR was performed under reaction conditions shown in Table 1, and an *Exicycler*^{™} 96 (Bioneer, A-2060) was used for real-time nucleic acid amplification reaction.

As shown in FIG. 4, the nucleic acid extraction method using the acidic solution composition exhibited virus nucleic acid extraction performance equivalent to or better than the method using ethanol.

### Example 4: Comparison of extraction methods using acidic proteolytic enzyme (hereafter referred to as acid protease) and proteinase K (viral nucleic acid extraction and real-time polymerase chain reaction)

Extraction efficiencies using acidic protease and proteinase K were compared. After purifying a viral nucleic acid using a lysis buffer including each of two proteolytic enzymes, real-time RT-PCR was performed.

As a sample, 40000 E-gene RNA positive materials of SARS-CoV-2 were used and diluted in DEPC-DW so that the total volume was 400 µl per test. Proteinase K and Poly(A) were added to a lysis buffer including a guanidine salt compound, and the lysis buffer including proteinase K was mixed with the solution including the positive materials. The mixture including proteinase K was dissolved at 60°C for 10 minutes. On the other hand, a lysis buffer was prepared by mixing acetic acid with a guanidine salt compound, acidic protease and Poly(A) were added thereto, and the lysis buffer including acidic protease was mixed with the solution including the positive materials. The mixture including acidic protease was dissolved at room temperature for 10 minutes.

The acetic acid solution and silica magnetic particles were added to the cell lysate for the sample including proteinase K, and only silica magnetic particles were added thereto for the sample including acidic protease, followed by mixing, so that the nucleic acid and the magnetic particles were allowed to bind to each other. After removing only the supernatant using a magnet, washing was performed once again using a low-concentration acidic solution (at a pH of 4.5-5.0). After removing the supernatant using a magnet, the silica magnetic particles to which the nucleic acid bound were dissolved in 100 µl of a Tris hydrochloride solution (at a pH of 9.0-10.0) to collect the nucleic acid.

The collected nucleic acid solution was added to an *AccuPower*^{®} SARS-CoV-2 Real-Time RT-PCR Kit and real-time reverse transcription polymerase chain reaction was performed. A tube for qPCR was prepared, 5 µl of the nucleic acid solution was added to a PCR mix of an *AccuPower*^{®} SARS-CoV-2 Real-Time RT-PCR Kit according to the protocol and mixed by pipetting, the upper surface of the diagnostic kit was completely sealed with an adhesive sealing film, and the diagnostic kit composition and the nucleic acid were thoroughly mixed using an *ExiSpin*^{™} (Bioneer, BS-010211-AAL). Here, real-time RT-PCR was performed under reaction conditions shown in Table 2 below, and an *Exicycler*^{™} 96 (Bioneer, A-2060) was used for real-time nucleic acid amplification reaction.

As shown in FIG. 5, the method of dissolving the sample using acidic protease exhibited viral nucleic acid extraction performance equivalent to the method using proteinase K.

**[Table 2]**

| Line | STEP | Temperature | Running Time |
|---|---|---|---|
| 1 | cDNA synthesis | 50°C | 30 min |
| 2 | Pre-denaturation | 94°C | 10 min |
| 3 | Denaturation | 95°C | 15 see |
| 4 | Annealing & Extension | 60°C | 1 min |
| 5 | Scan | | |
| 6 | Go to line 3, 45 cycles | | |
| 7 | End | | |

Having described specific parts of the present invention in detail above, it will be obvious to those skilled in the art that these specific descriptions are only preferred embodiments, and the scope of the present invention is not limited thereby. Accordingly, the substantial scope of the present invention will be defined by the appended claims and equivalents thereto.

## Claims

1. A method of isolating a nucleic acid using a difference in pH between buffers, comprising:
(a) binding a nucleic acid to silica magnetic particles by adding silica magnetic particles to a sample containing a nucleic acid, a mixture of binding buffer comprising 0.175 M or more aqueous acetic acid solution or 2.18 M or less aqueous phosphoric acid solution;
(b) washing the magnetic particles to which the nucleic acid binds in an acidic pH buffer; and
(c) separating the magnetic particles and eluting the nucleic acid in a basic pH elution buffer.

2. The method of isolating a nucleic acid of claim 1, wherein an alcohol precipitation method is not used.

3. The method of isolating a nucleic acid of claim 1, wherein the magnetic particles have a size of 50 nm to 1 µm and are coated with a material containing a siliceous material selected from the group consisting of silica, quartz, and silicon.

4. The method of isolating a nucleic acid of claim 1, wherein the nucleic acid is DNA or RNA, and a change in pH of the binding buffer, the acidic pH buffer, and the basic pH elution buffer is used.

5. The method of isolating a nucleic acid of claim 1, wherein steps (b) and (c) comprise applying a magnetic field using a magnet.

6. The method of isolating a nucleic acid of claim 1, wherein step (a) comprises mixing the sample comprising the nucleic acid with an acidic buffer at a pH of 1 to 6 and adding the magnetic particles to bind the nucleic acid to the magnetic particles.

7. The method of isolating a nucleic acid of claim 1, wherein step (b) comprises attaching the magnetic particles using a magnet, removing a supernatant, and washing with an acidic solution at a pH of 4 to 6.5.

8. The method of isolating a nucleic acid of claim 1, wherein step (c) comprises removing a supernatant using a magnet and dissolving the magnetic particles to which the nucleic acid binds in a basic solution at a pH of 8.0 to 10.0 to collect the nucleic acid.

9. The method of isolating a nucleic acid of claim 1, wherein the mixture of binding buffer further comprises an acidic protease or proteinase K.
